# EUROPEAN PATENT SPECIFICATION

(11) **EP 3 674 710 B1**
(45) Date of publication and mention of the grant of the patent: **28.02.2024**
(21) Application number: 18848723.5
(22) Date of filing: 20.08.2018
(51) Int. Cl.: G01N 33/68, C12Q 1/6883

(54) **LIVER DISEASE PREDICTION OR DIAGNOSIS METHOD FOR PATIENTS WITH NAFLD.**
LEBERERKRANKUNGSVORHERSAGE- ODER -DIAGNOSEVERFAHREN FÜR PATIENTEN MIT NAFLD
MÉTHODE DE PRÉDICTION OU DE DIAGNOSTIC D'HÉPATOPATHIE POUR LES PATIENTS AVEC NAFLD

(30) Priority: 21.08.2017 KR 20170105714
(43) Date of publication of application: 01.07.2020
(73) Proprietor: Seoul National University Hospital, Seoul 03080 (KR)
(72) Inventor: KIM, Won, Seoul 06601 (KR)
(74) Representative: Plougmann Vingtoft a/s
(86) International application number: PCT/KR2018/009548
(87) International publication number: WO 2019/039817

(56) References cited:
- WO-A1-2017/132673
- JP-A- 2012 515 335
- JP-A- 2012 515 336
- EAUM SEOK LEE ET AL: "Growth Differentiation Factor 15 Predicts Chronic Liver Disease Severity", GUT AND LIVER, vol. 11, no. 2, 15 March 2017 (2017-03-15) , pages 276-282, XP55615812, KR ISSN: 1976-2283, DOI: 10.5009/gnl16049
- LEE, E. S. et al.: "Growth Differentiation Factor 15 Predict Chronic Liver Disease Severity", Gut and Liver, vol. 11, no. 2, 13 October 2016 (2016-10-13), pages 276-282, XP55615812,
- LIU, X. et al.: "Association of Serum Level of Growth Differentiation Factor 15 with Liver Cirrhosis and Hepatocellular Carcinoma", PLoS ONE, vol. 10, no. 5, 2015, page e0127518, XP55615813,
- HALIM, M. H. et al.: "Significance of Growth Differentiation Factor 15 in Chronic HCV Patient", Journal of Genetic Engineering and Biotechnology, vol. 15, 12 August 2017 (2017-08-12), pages 403-407, XP55615825,

## Description

### Technical Field

The present invention relates to a method for prediction or diagnosis of a liver disease and, more specifically, to the use of a marker for prediction or diagnosis of liver fibrosis in non-alcoholic fatty liver disease in a method for prediction or diagnosis using the same.

### Background Art

Growth differentiation factor 15 (GDF15) is widely distributed in mammalian tissues and has been known to play multiple roles in inflammation, cancer, and cardiovascular diseases. In recent years, serum GDF15 levels were found to increase in patients with liver cirrhosis and hepatocellular carcinoma associated with chronic hepatitis B or C virus infection. Although the roles in the mechanism of GDF15 in liver diseases are not clear, GDF15 has been revealed not only to stimulate the expression of transforming growth factor beta 1 (TGF-β1) but also to directly activate the SMAD signal system which plays an important role in liver fibrosis/carcinogenesis pathways.

It has been recently reported that sarcopenia is an independent risk factor for non-alcoholic steatohepatitis (NASH) and non-alcoholic fatty liver disease (NAFLD). Since GDF15 expression is up-regulated in muscle-wasting conditions, serum GDF15 levels might influence the histological severity of DAFLD through the control of muscle mass.

Accordingly, the pathogenic roles of GDF15 in the development and progression of NAFLD need to be established by investigating whether GDF15 increases the risk of NASH development and advanced fibrosis among biopsy-proven NAFLD patients, independently from known metabolic risk factors, and whether the exposure of hepatocytes to a high concentration of GDF15 influences liver fibrosis.

Prior art by EAUM SEOK LEE ET AL: "Growth Differentiation Factor 15 Predicts Chronic Liver Disease Severity", GUT AND LIVER, vol. 11, no. 2, 15 March 2017, pages 276-282, discloses experimental evidence for the association of the levels of GDF15 and fibrosis in patients with liver diseases.

### Detailed Description of the Invention

### Technical Problem

The present inventors endeavored to develop a marker for prediction or diagnosis of liver fibrosis in non-alcoholic fatty liver disease (NAFLD) and a prediction or diagnosis method using the same.

As a result, the present inventors established that there is a positive correlation between the growth differentiation factor 15 (GDF15) protein level and liver fibrosis, and thus completed the present invention.

Therefore, an aspect of the present invention is to provide the use of a composition in a method for prediction or diagnosis of a liver disease, the composition comprising an agent for measuring the expression of a nucleic acid sequence encoding a growth differentiation factor 15 (GDF15) protein or the activity of the GDF15 protein.

Another aspect of the present invention is to provide a method for prediction or diagnosis of a liver disease, the method comprising a measurement step of measuring the expression of a nucleic acid sequence encoding a GDF15 protein or the concentration of the GDF15 protein in a sample.

Still another aspect of the present invention is to provide a method for screening a candidate substance for prevention, treatment, or alleviation of a liver disease, the method comprising:
a treatment step of treating an analysis sample with a GDF15 protein; and
an analysis step of selecting an analysis sample inhibiting the activity of the GDF15 protein.

Still another aspect of the present invention is to provide a method for screening a candidate substance for prevention, treatment, or alleviation of a liver disease, the method including:
a treatment step of treating an analysis sample with GDF15 protein-expressing cells; and
an analysis step of selecting an analysis sample inhibiting the activity of GDF15 protein in the cells.

### Technical Solution

The present invention relates to the use of a composition comprising an agent for measuring the expression of nucleic acid sequence encoding GDF15 protein or the activity of GDF15 protein,in a method for prediction or diagnosis of a liver disease and a method for prediction or diagnosis of a liver disease using the same, and more specifically, the present invention includes the use of a marker in a method for prediction or diagnosis of liver fibrosis in non-alcoholic fatty liver disease (NAFLD).

Hereinafter, the present invention will be described in detail.

In accordance with an aspect of the present invention, there is provided a use of a composition in a method for prediction or diagnosis of a liver disease, the composition comprising an agent for measuring the expression of a nucleic acid sequence encoding a growth differentiation factor 15 (GDF15) protein or the activity of the GDF15 protein.

The liver disease is liver fibrosis in non-alcoholic fatty liver disease.

The GDF15 protein may comprise the amino acid sequence of SEQ ID NO: 1 and, for example, may consist of the amino acid sequence of SEQ ID NO: 1.

The agent may be an antibody capable of specifically binding to a GDF15 protein or a fragment thereof, but is not limited thereto.

The agent may further comprise a detector, which specifically binds to an antibody capable of specifically binding to the GDF15 protein or a fragment thereof.

The detector may be: a conjugate labeled with a chromogenic enzyme, a fluorescent substance, a radioactive isotope, or a colloid; or a secondary antibody capable of specifically binding to an antibody capable of specifically binding to the GDF15 protein or a fragment thereof, but is not limited thereto.

The GDF15 protein may be derived from a biological sample isolated from a subject.

The biological sample may be obtained from blood or biopsy tissue, but is not limited thereto.

In accordance with the present invention, there is provided a method for prediction or diagnosis of a liver disease, the method comprising a measurement step of measuring the expression of a nucleic acid sequence encoding a growth differentiation factor 15 (GDF15) protein or the concentration of the GDF15 protein in a sample.

The liver disease is liver fibrosis in non-alcoholic fatty liver disease

The GDF15 protein may comprise the amino acid sequence of SEQ ID NO: 1 and, for example, may consist of the amino acid sequence of SEQ ID NO: 1.

The GDF15 protein may be derived from a biological sample isolated from a subject.

The biological sample may be obtained from blood or biopsy, but is not limited thereto.

The measurement step may be performed using any one selected from the group consisting of enzyme-linked immunosorbent assay (ELISA), a colorimetric method, an electrochemical method, a fluorimetric method, luminometry, a particle counting method, visual assessment, a scintillation counting method, and immunohistochemical staining, and for example, may be performed using ELISA, but is not limited thereto.

In the measurement step, whether the concentration of the GDF15 protein is 1.52 ng/mL or more may be determined.

In an example of the present invention, GDF15 levels were classified into four quartiles (Q). Q4 is the highest quartile of GDF15 levels, indicating that the concentration of GDF15 protein was 1.52 ng/mL or more, and in such a case, the prevalence of advanced fibrosis was 41.7%.

The GDF15 levels corresponding to Q4 may be considered to be associated with advanced fibrosis, and therefore, GDF15 can be utilized as a marker for predicting or diagnosing liver fibrosis in non-alcoholic fatty liver disease (NAFLD) by using a method for determining whether the concentration of GDF15 protein is 1.52 ng/ml or more.

In accordance with a still another aspect of the present invention, there is provided a method for screening a candidate substance for prevention, treatment, or alleviation of a liver disease, the method comprising:
a treatment step of treating an analysis sample with a growth differentiation factor 15 (GDF15) protein; and
an analysis step of selecting an analysis sample inhibiting the activity of the GDF15 protein.

The liver disease is liver fibrosis in non-alcoholic fatty liver disease.

The analysis sample may be obtained from blood or biopsy, but is not limited thereto.

The analysis step may be performed to measure the activity of GDF15 protein by using a method selected from the group consisting of SDS-PAGE, immunofluorescent assay, enzyme-linked immunosorbent assay (ELISA), mass spectrometry, and protein chip assay, but is not limited thereto.

The GDF15 protein may include the amino acid sequence of SEQ ID NO: 1 and, for example, may consist of the amino acid sequence of SEQ ID NO: 1

In accordance with a still another aspect of the present invention, there is provided a method for screening a candidate substance for prevention, treatment, or alleviation of a liver disease, the method including:
a treatment step of treating an analysis sample with growth differentiation factor 15 (GDF15) protein-expressing cells; and
an analysis step of selecting an analysis sample inhibiting the activity of GDF15 protein in the cells.

The liver disease is liver fibrosis in non-alcoholic fatty liver disease.

The analysis sample may be obtained from blood or biopsy, but is not limited thereto.

The measurement step may be performed, in order to measure the expression of the GDF15 protein, by using a method selected from the group consisting of western blotting, enzyme-linked immunosorbent assay (ELISA), immunohistochemical staining, immunoprecipitation, and immunofluorescence, but is not limited thereto.

The GDF15 protein may include the amino acid sequence of SEQ ID NO: 1 and, for example, may consist of the amino acid sequence of SEQ ID NO: 1.

### Advantageous Effects

The present invention is directed to the use of a composition in a method for prediction or diagnosis of a liver disease and a method for prediction or diagnosis of a liver disease using the same, wherein growth differentiation factor 15 (GDF15) protein levels, which are positively correlated with liver fibrosis in non-alcoholic fatty liver disease (NAFLD), can be checked through the use of the composition comprising an agent for measuring the expression of nucleic acid sequence encoding GDF15 protein or the activity of GDF15 protein and thus GDF15 can be effectively used in the prediction and diagnosis of liver fibrosis in NAFLD.

### Brief Description of the Drawings

FIG. 1A is a graph showing that growth differentiation factor 15 (GDF15) levels increased significantly with the histological severity of non-alcoholic fatty liver disease (NAFLD).
FIG. 1B is a graph showing mean GDF15 levels according to steatosis grades.
FIG. 1C is a graph showing mean GDF15 levels according to ballooning grades.
FIG. 1D is a graph showing mean GDF15 levels according to lobular inflammation grades.
FIG. 1E is a graph showing mean GDF15 levels according to fibrosis stages.
FIG. 1F is a graph showing mean GDF15 levels according to the fibrosis stage severity.
FIG. 2A is an image showing liver tissue corresponding to fibrosis stage F0, the liver tissue being stained with Masson's trichrome.
FIG. 2B is an image showing liver tissue corresponding to fibrosis stage F0, the liver tissue being stained with anti-GDF15 antibody.
FIG. 2C is an image showing liver tissue corresponding to fibrosis stage F1, the liver tissue being stained with Masson's trichrome.
FIG. 2D is an image showing liver tissue corresponding to fibrosis stage F1, the liver tissue being stained with anti-GDF15 antibody.
FIG. 2E is an image showing liver tissue corresponding to fibrosis stage F2, the liver tissue being stained with Masson's trichrome.
FIG. 2F is an image showing liver tissue corresponding to fibrosis stage F2, the liver tissue being stained with anti-GDF15 antibody.
FIG. 2G is an image showing liver tissue corresponding to fibrosis stage F3, the liver tissue being stained with Masson's trichrome.
FIG. 2H is an image showing liver tissue corresponding to fibrosis stage F3, the liver tissue being stained with anti-GDF15 antibody.
FIG. 3A is a graph showing a correlation between the GDF15 level and liver stiffness.
FIG. 3B is a graph showing advanced fibrosis verified according to the serum GDF15 level and diabetes status.
FIG. 4A is an immuno-blotting image of α-smooth muscle actin (α-SMA) over time after an extract of LX-2 cells, which are human hepatic stellate cells (HSCs), was treated with recombinant human GDF15 (rhGDF15).
FIG. 4B provides immunofluorescent staining images of α-SMA over time after LX-2 cells were treated with rhGDF15.
FIG. 4C is an immuno-blotting image of α-SMA over time after LX-2 cells were treated with rhGDF15.
FIG. 4D is an immuno-blotting image of α-SMA over time after an extract of hepatocytes was treated with rhGDF15.
FIG. 5A is a graph showing expression levels of GDF15 mRNA after 3 hours of hepatocytes, HSCs, and Kupffer cells were subjected to palmitate treatment.
FIG. 5B is a graph showing expression levels of GDF15 mRNA after 6 hours of hepatocytes, HSCs, and Kupffer cells were subjected to palmitate treatment.

### Best Mode for Carrying Out the Invention

The present invention is directed to the use of a composition in a method for prediction or diagnosis of a liver disease, the composition comprising an agent for measuring the expression of a nucleic acid sequence encoding a growth differentiation factor 15 (GDF15) protein or the activity of the GDF15 protein.

### Mode for Carrying Out the Invention

Hereinafter, the present invention will be described in more detail with reference to examples. These examples are only for illustrating the present invention, and it would be obvious to those skilled in the art that the scope of the present invention is not construed as being limited to the examples, but rather is defined by the appended claims.

### Test Example 1: Subject selection

Subjects were selected based on whether or not they had a radiological evidence of hepatic steatosis.

The eligibility criteria for subjects were as follows:
(i) at least 18 years old;
(ii) bright echogenic liver on ultrasound scanning (increased liver/kidney ecogenicity and posterior attenuation); and
(iii) unexplained elevation of alanine transaminase (ALT) levels above the reference value within 6 months.

The following exclusion criteria were applied:
(i) hepatitis B or C virus infection;
(ii) autoimmune hepatitis;
(iii) drug-induced liver injury or steatosis;
(iv) Wilson disease or haemochromatosis;
(v) excessive alcohol consumption (male > 30 g/day, female > 20 g/day); and
(vi) malignancy diagnosis.

A test group included patients who underwent live biopsy for suspected nonalcoholic steatohepatitis (NASH) or fibrosis.

In addition, a control group was established to include sera collected in a liver biopsy of liver tissues and a pre-evaluation for donor liver transplantation or sera collected for characterization of solid liver mass suspected of hepatic adenoma or focal nodular hyperplasia without any evidence of hepatic steatosis, on the basis of radiological results.

### Test Example 2: Measurement

A body mass index (BMI, ≥ 25 kg/m²) was used as a criterion for obesity on the basis of the World Health Organization Asia-Pacific criteria.

Metabolic syndrome was defined on the basis of the revised National Cholesterol Education Program Adult Treatment Panel III criteria.

The insulin resistance was evaluated for venous blood samples having undergone fast biopsy for 12 hours (overnight) using the homeostasis model assessment of insulin resistance (HOMA-IR).

Sarcopenia was evaluated by appendicular skeletal muscle mass (ASM) divided by body weight (ASM/weight, ASM%).

Low skeletal muscle mass (LSMM) was defined as appendicular skeletal muscle mass (ASM) divided by BMI (ASM/BMI) according to the Foundation for the National Institutes of Health Sarcopenia Project.

Growth differentiation factor 15 (GDF15) levels were measured using a commercially available enzyme-linked immunosorbent assay kit (ELISA; R&D Systems, Minneapolis, MN). The amino acid sequence information of GDF15 is shown in Table 1 below.

**[Table 1]**

| SEQ ID NO. | Name | Sequence |
|---|---|---|
| 1 | GDF15 | ARARNGDHCPLGPGRCCRLHTVRASLEDLGWADWVLS PREVQVTMCIGACPSQFRAANMHAQIKTSLHRLKPDT VPAPCCVPASYNPMVLIQKTDTGVSLQTYDDLLAKDC HCI |

For measurement of liver stiffness, transient elastography (TE) was performed to predict advanced fibrosis. The NAFLD fibrosis score (NFS), Fibrosis-4 (Fib4) index, and aspartate aminotransferase (AST) to platelet ratio index (APRI) were calculated to compare these indices against GDF15 in view of diagnostic performance for predicting advanced fibrosis.

### Test Example 3: Liver histological evaluation

Non-alcoholic fatty liver disease (NAFLD) was diagnosed according to the presence or absence of 5% or more macrovesicular steatosis. NASH was diagnosed based on an overall pattern of histological hepatic injury in the form of macrovesicular steatosis. Fibrosis was assessed according to such criteria. Advanced fibrosis was defined only at the stage of F3 or higher.

### Test Example 4: Liver cell culture in presence of GDF15

Recombinant human GDF15 (rhGDF15; R&D Systems; 957-GD-025) was added to LX-2 cells (human hepatic stellate cells, KAIST), cultured in the presence of 100 ng/mL GDF15 and 10 ng/mL TGF-β, and primary hepatocytes, directly isolated from the mouse liver, at 100 mg/ml per 3×10⁵ cells/6 wells. The levels of fibrosis markers, such as alpha-smooth muscle actin (α-SMA) and collagen 1, were assessed by immunoblotting, immunohistochemistry, and immunofluorescence staining.

### Test Example 5: Assessment of GDF15 mRNA expression level change through palmitate treatment

Primary hepatic cells were isolated and differentiated into hepatocytes, HSCs, Kupffer cells, and liver sinusoidal endothelial cells (LSECs). Kupffer cells are macrophages present in the liver, and are known to play an essential role in the initiation of inflammation.

To distinguish Kupffer cells and LSECs, magnetic-activated cell sorting (MACS) was performed using the MiniMACS^{™} separator system (MiltenyiBiotec, Seoul, Korea). The purity of LSECs was evaluated by fluorescence-activated cell sorting using anti-PE antibody (purity of 90% or higher).

Primary cells were cultured in 1.5 mL of a serum-free medium for 4 hours, and treated with 5(w/w)% of bovine serum albumin (BSA, control), 200 µM palmitate/ 5(w/w)% BSA solution, and 500 µM palmitate / 5(w/w)% BSA, respectively.

After 3 and 6 hours, cells were collected and further analyzed. Reverse-transcription polymerase chain reaction (RT-PCR) and real-time PCR were performed to assess the expression of GDF15 mRNA after palmitate treatment.

### Test Example 6: Statistical analysis

Between-group differences were evaluated using the independent t test, Mann-Whitney U test, analysis of variance (ANOVA), or Kruskal-Wallis test for continuous variables, and the chi-square test for categorical variables. Spearman's correlation analysis was performed to assess the relationship between GDF15 levels and histological parameters.

A general linear model adjusted for age, gender, and insulin resistance (homeostasis model assessment of insulin resistance, HOMA-IR), was used to compare GDF15 levels according to NASH or advanced fibrosis status.

To investigate the independent prediction factors of NASH or fibrosis, a binary logistic regression model adjusted for covariates was created. Significance was defined as P <0.05.

All statistical analyses were performed using IBM SPSS statistics software version 20.0 (IBM Inc., Armonk, NY) .

### Result Example 1: Clinical characteristics according to histological spectrum of NAFLD

As shown in Table 2, 150 NAFLD patients and 40 control subjects were explored. The NAFLD patients were classified into 72 non-alcoholic fatty liver patients (male, 65.3%) and 78 NASH patients (male, 44.9%) through biopsy.

**[Table 2]**

| | Control | NAFL | NASH | P-value³ |
|---|---|---|---|---|
| N | 40 | 72 | 78 | |
| Male, n (%) | 17 (42.5) | 47 (65.3) | 35 (44.9) | 0.732 |
| Age, year | 54.1 ± 13.9 | 53.0 ± 12.1 | 52.9 ± 16.2 | 0.903 |
| Body mass index (BMI), kg/m² | 24.8 (23.4, 26.3) | 27.4 (25.0, 29.5) | 28.4 (25.8, 31.6) | <0.001 |
| Systolic blood pressure (SBP), mmHg | 123.2 ± 12.9 | 126.4 ± 13.7 | 128.8 ± 17.0 | 0.154 |
| Diastolic blood pressure (DBP), mmHg | 77.2 ±7.4 | 79.1 ± 11.4 | 79.6 ± 12.5 | 0.529 |
| Waist circumference (WC), cm | 87.1 (82.0, 93.5) | 92.0 (87.6, 99.6) | 95.1 (89.1, 103.6) | <0.001 |
| Diabetes, n (%) | 5 (12.5) | 25 (34.7) | 32 (41.0) | 0.003 |
| Hypertension, n (%) | 15 (37.5) | 33 (45.8) | 46 (59.0) | 0.021 |
| Metabolic syndrome, n (%) | 14 (35.0) | 45 (65.2) | 61 (79.2) | <0.001 |
| Obesity¹, n (%) | 16 (40.0) | 55 (76.4) | 65 (83.3) | <0.001 |
| LSMM², n (%) | 2 (5.0) | 13 (18.6) | 26 (34.2) | <0.001 |
| ALT, IU/L | 22.5 (13.3, 34.8) | 28.0 (22.0, 45.8) | 59.5 (36.8, 108.0) | <0.001 |
| AST, IU/L | 25.0 (19.3, 37.0) | 28.5 (22.0, 34.8) | 54.0 (35.8, 80.3) | <0.001 |
| Platelet (Plt), ×10⁹/L | 228.4 ± 44.7 | 242.5 ± 62.3 | 218.6 ± 61.1 | 0.046 |
| Albumin, mg/dL | 4.1 ± 0.4 | 4.2 ± 0.3 | 4.3 ± 0.3 | 0.022 |
| HbA1c, % | 5.7 (5.4, 5.8) | 5.8 (5.4, 6.5) | 6.2 (5.6, 7.0) | <0.001 |
| Triglyceride, mg/dL | 94.0 (71.3, 127.0) | 145.5 (106.5, 192.0) | 142.0 (107.0, 191.8) | <0.001 |
| LDL cholesterol, mg/dL | 109.3 ± 34.0 | 103.7 ± 33.6 | 108.2 ± 30.3 | 0.602 |
| ASM/BMI | 0.73(0.60, | 0.81(0.64, | 0.67(0.55, | 0.005 |
| | 0.89) | 0.90) | 0.81) | |
| Insulin, µIU/mL | 7.8 (6.2, 9.6) | 10.4 (7.9, 13.2) | 15.7 (11.0, 25.2) | <0.001 |
| Insulin resistance (HOMA-IR) | 2.05 (1.69, 2.54) | 2.62 (1.97, 3.55) | 4.30 (2.95, 7.86) | <0.001 |
| GDF15, ng/mL | 0.71 (0.41, 1.22) | 0.67 (0.43, 1.07) | 1.1 (0.65, 1.83) | 0.002 |
| Fibrosis stage | | | | |
| 0 | 23 (57.5) | 24 (33.3) | 4 (5.1) | <0.001 |
| 1 | 13 (32.5) | 42 (58.3) | 24 (30.8) | |
| 2 | 2 (5.0) | 2 (2.8) | 27 (34.6) | |
| 3 | 1 (2.5) | 4 (5.6) | 9 (11.5) | |
| 4 | 1 (2.5) | 0 | 14 (17.9) | |

As can be seen from Table 2 above, the present inventors found a linear correlation between NAFLD severity and BMI, waist circumference, relevant conditions (diabetes, hypertension, and maculopathy), alanine transaminase (ALT) and aspartate transaminase (AST) levels, and insulin resistance (HOMA-IR) (¹BMI ≥ 25kg/m², ²ASM/BMI < 0.789 in men and < 0.512 in women according to the Foundation for the National Institutes of Health Sarcopenia Project, ³independent t-test or Mann-Whitney analysis test for continuous variables; chi-square test for categorical variables).

As can be confirmed from Table 3, among NAFLD patients (n = 150), patients with advanced fibrosis were older, more likely to be female, and had significantly lower serum albumin levels and platelet counts than the values noted among NAFLD patients without advanced fibrosis.

**[Table 3]**

| | F0-2 | F3-4 | P-value³ |
|---|---|---|---|
| N | 123 | 27 | |
| Male, n (%) | 73 (59.3) | 9 (33.3) | 0.014 |
| Age, year | 50.7 ± 14.2 | 63.1 ± 9.9 | <0.001 |
| Body mass index | 27.7 (25.2, | 27.4 (25.6, | 0.959 |
| (BMI), kg/m² | 30.6) | 30.0) | |
| Systolic blood pressure (SBP), mmHg | 127.3 ± 15.6 | 129.2 ± 15.2 | 0.561 |
| Diastolic blood pressure (DBP), mmHg | 80.0 ± 12.1 | 76.4 ± 11.0 | 0.156 |
| Waist circumference (WC), cm | 92.8 (88.6, 100.5) | 93.8 (89.0, 102.7) | 0.459 |
| Diabetes, n (%) | 40 (32.5) | 17 (63.0) | 0.003 |
| Hypertension, n (%) | 57 (46.3) | 22 (81.5) | 0.001 |
| Metabolic syndrome, n (%) | 85 (70.8) | 21 (80.3) | 0.303 |
| Obesity¹, n (%) | 98 (79.7) | 22 (81.5) | 0.832 |
| LSMM², n (%) | 30 (25.0) | 9 (34.6) | 0.315 |
| ALT, IU/L | 40.0 (25.0, 75.0) | 46.0 (28.0, 85.0) | 0.492 |
| AST, IU/L | 34.0 (25.0, 58.0) | 53.0 (35.0, 75.0) | 0.003 |
| Platelets (Plt), ×10⁹/L | 239.0 ± 60.3 | 189.2 ± 57.7 | <0.001 |
| Albumin, mg/dL | 4.3 ± 0.3 | 4.1 ± 0.2 | 0.028 |
| HbA1c, % | 6.0 (5.5, 6.4) | 6.7 (5.6, 7.8) | 0.005 |
| Triglyceride, mg/dL | 144.0 (112.0, 192.0) | 121.0 (82.0, 197.0) | 0.136 |
| LDL cholesterol, mg/dL | 108.6 ± 32.2 | 94.2 ±28.2 | 0.037 |
| ASM/BMI | 0.77 (0.61, 0.88) | 0.61 (0.54, 0.76) | 0.008 |
| Insulin, µIU/mL | 11.3 (8.7, 15.6) | 19.9 (12.5, 24.5) | <0.001 |
| Insulin resistance (HOMA-IR) | 2.97 (2.21, 4.35) | 5.10 (3.98, 7.91) | <0.001 |
| GDF15, ng/mL | 0.73 (0.52, 1.24) | 1.81 (1.01, 2.17) | <0.001 |

Advanced fibrosis showed a significant correlation with diabetes, hypertension, higher insulin resistance, and lower ASM/BMI (P: 0.003, 0.001, <0.001, 0.008), but not with waist circumference or BMI (¹BMI ≥25 kg/m², ²ASM/BMI < 0.789 in men and < 0.512 in women according to the Foundation for the National Institutes of Health Sarcopenia Project, ³ANOVA or Kruskal-Wallis test for continuous variables; chi-square test for categorical variables).

### Result Example 2: Relation between GDF15 levels and advanced fibrosis

As shown in Table 2 above, the GDF15 levels significantly increased with the histological severity of NAFLD.

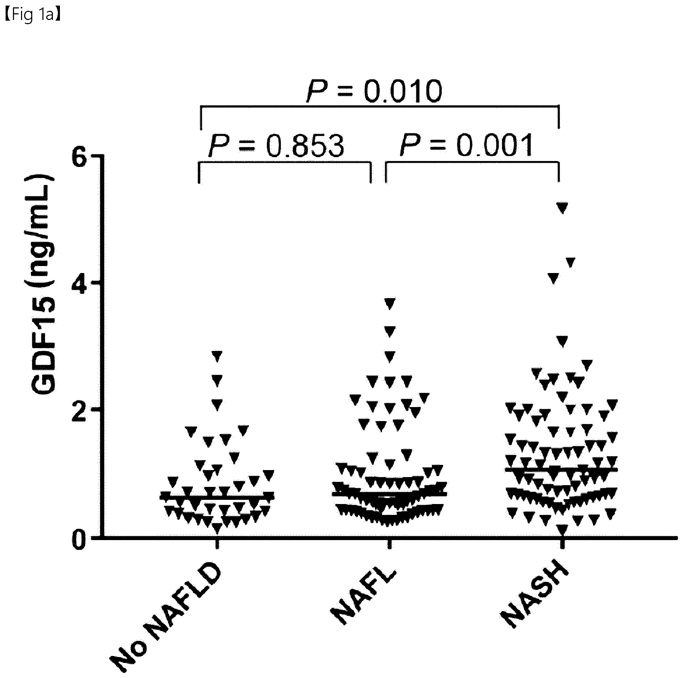

As can be confirmed in FIG. 1A, NASH patients showed significantly higher GDF15 levels than those shown in controls or NAFL patients.

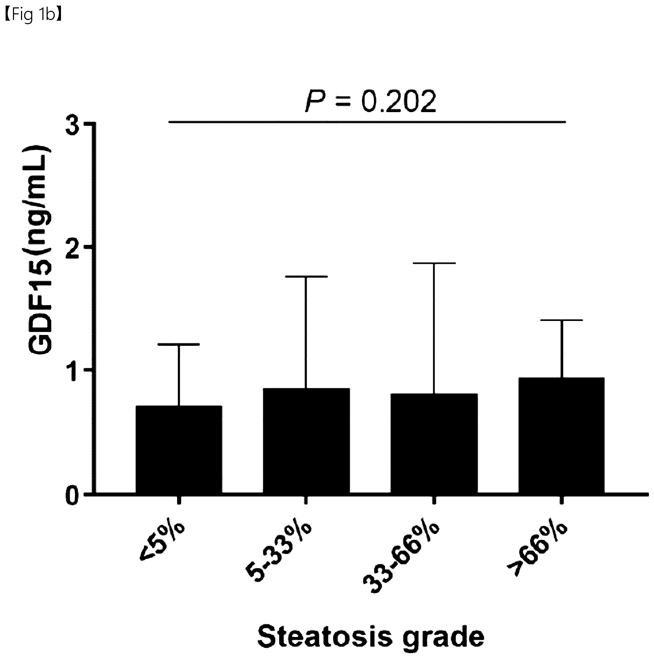

On the other hand, as can be confirmed in FIG. 1B, there was no association between GDF15 levels and steatosis grades (P = 0.202).

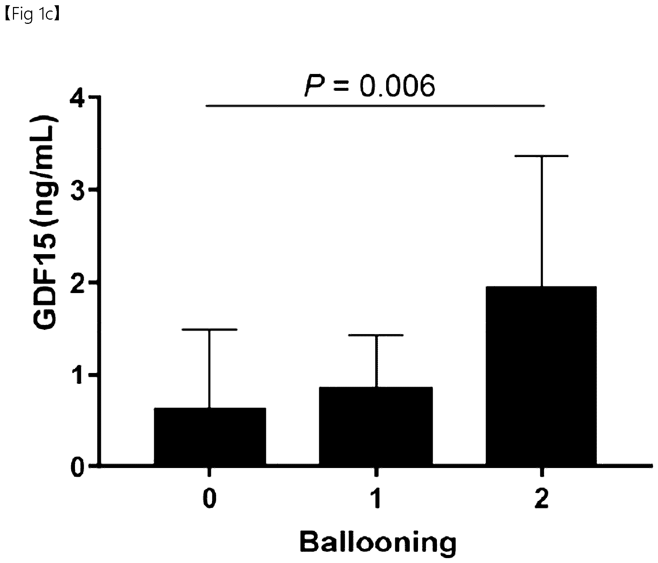

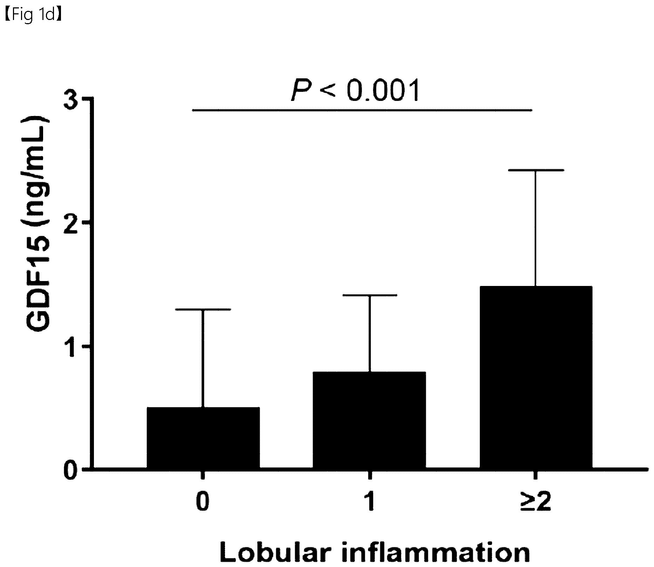

As can be confirmed in FIGS. 1C and 1D, serum GDF15 levels increased significantly with the severity of ballooning (Spearman's ρ, 0.200; P = 0.006) and lobular inflammation (Spearman's ρ, 0.271; P <0.001).

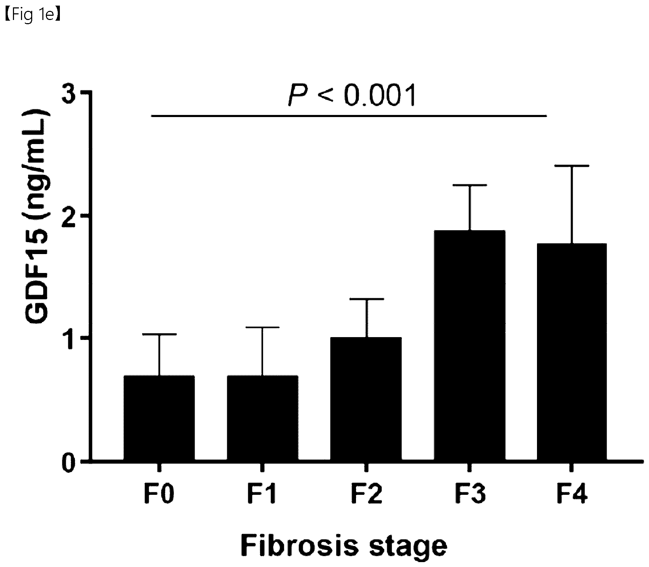

As can be confirmed in FIG. 1E, the degree of fibrosis was significantly correlated with serum GDF15 levels (Spearman's ρ, 0.337; P <0.001).

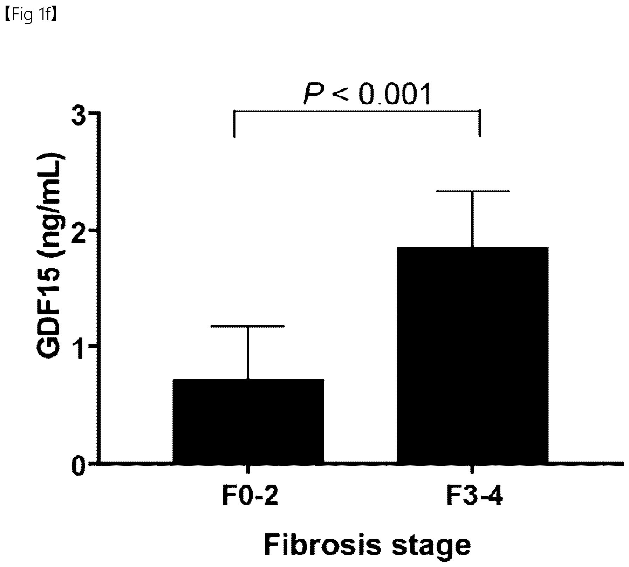

As can be confirmed in FIG. 1F, F3 and F4, that is, subjects with advanced fibrosis, showed significantly higher GDF15 levels compared with F0 to F2, that is, those without (P <0.001).

As a result, it can be verified that GDF15 levels have a stepwise relationship with the histological severity of NAFLD and a positive correlation with the severity of lobular inflammation, ballooning, and fibrosis.

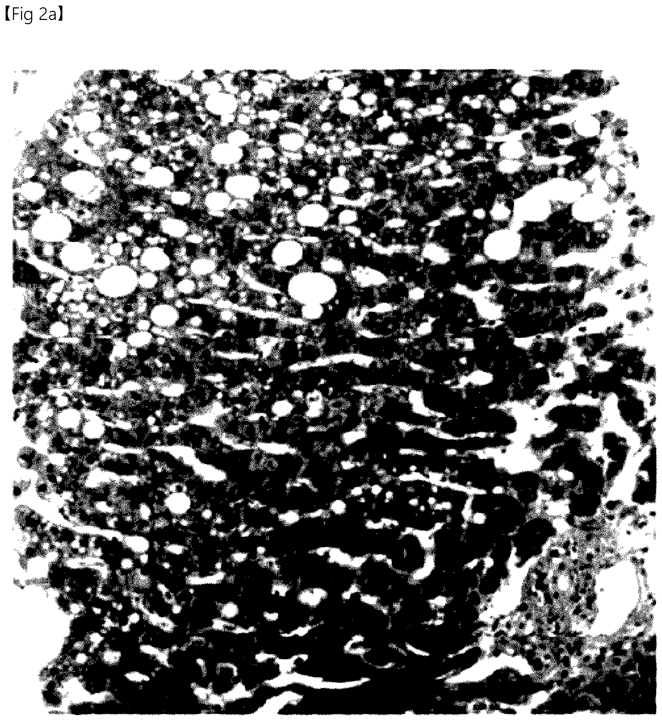

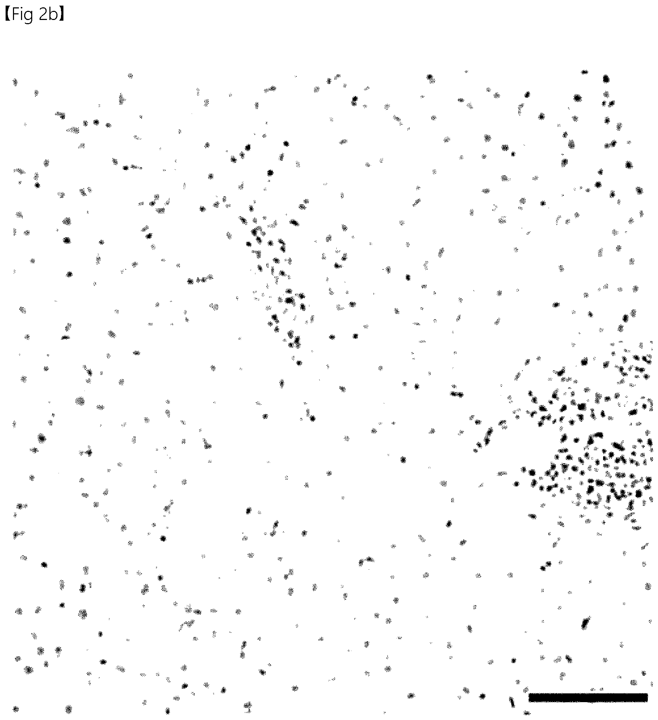

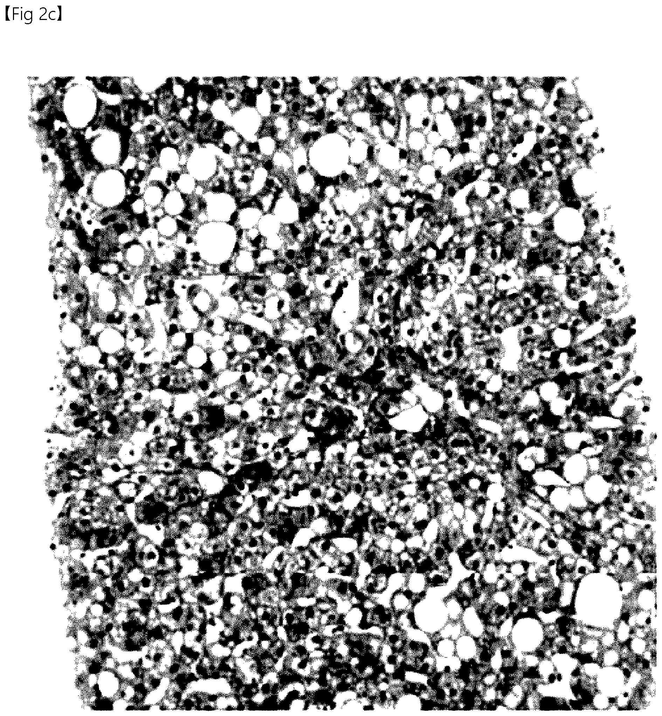

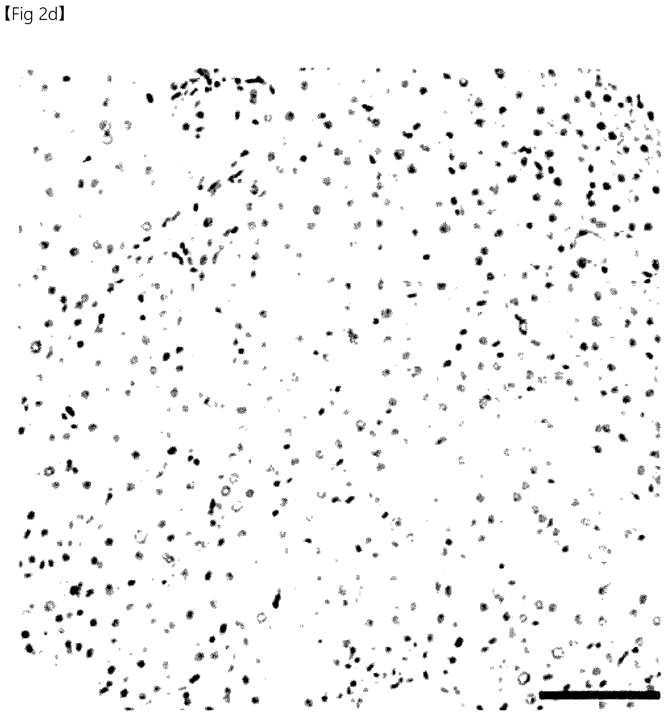

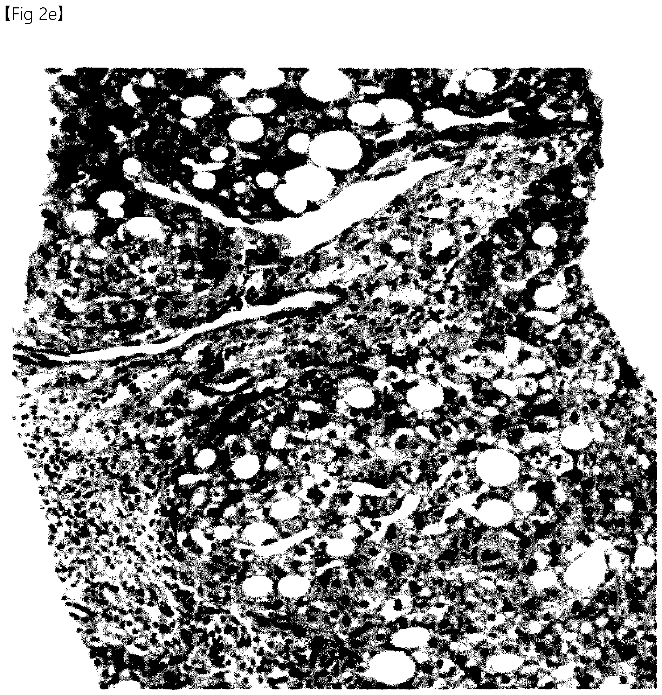

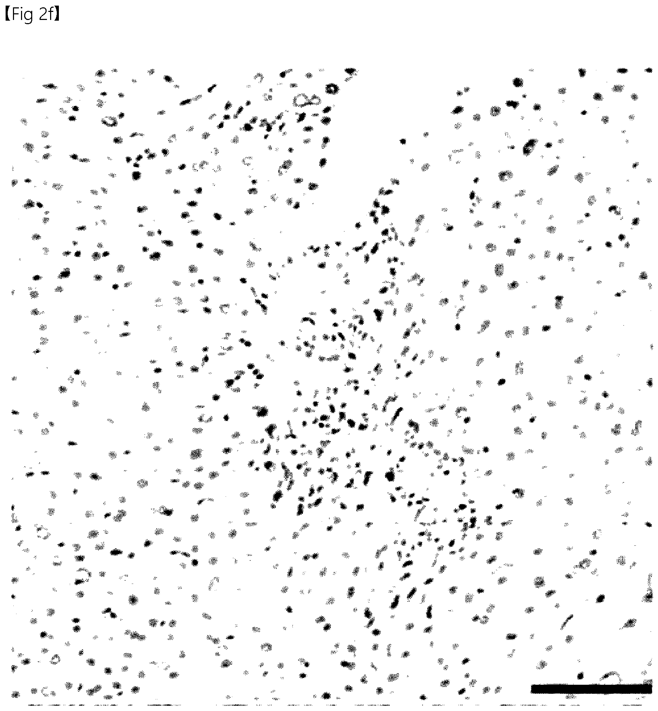

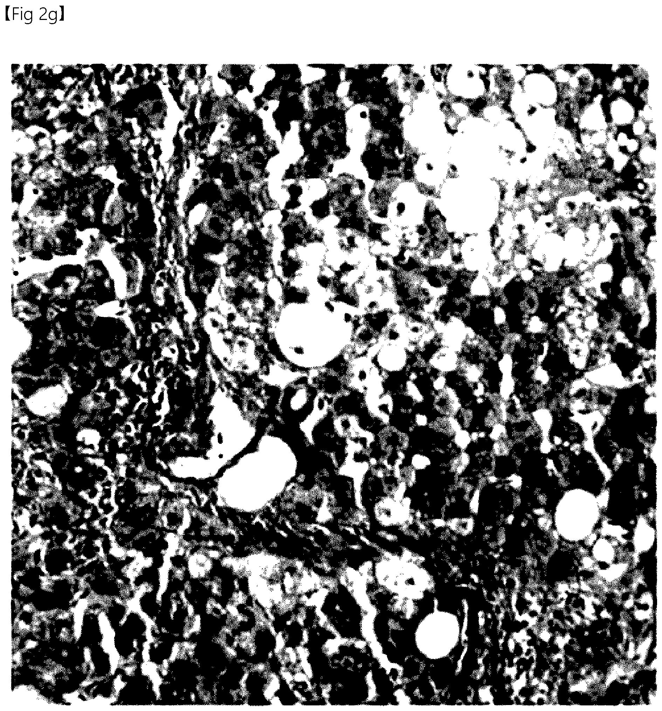

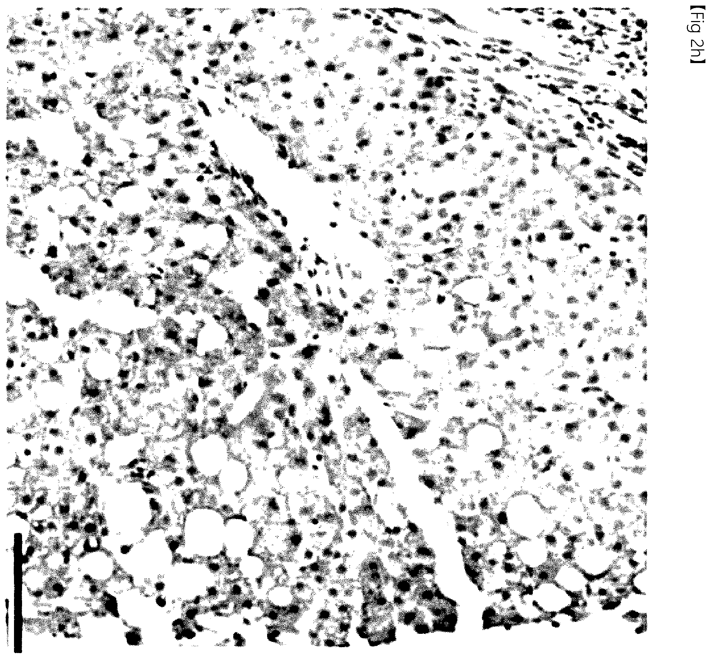

As can be confirmed in FIG. 2, immunohistochemical analysis confirmed that hepatic GDF15 expression levels were markedly higher in the subjects with advanced fibrosis shown in FIG. 2H than in subjects without advanced fibrosis shown in FIGS. 2B, 2D, and 2F.

Since the GDF15 levels were independently associated with advanced fibrosis (≥F3), it was investigated whether there was a correlation between GDF15 levels and TE values indicating fibrosis severity and expressed as liver stiffness.

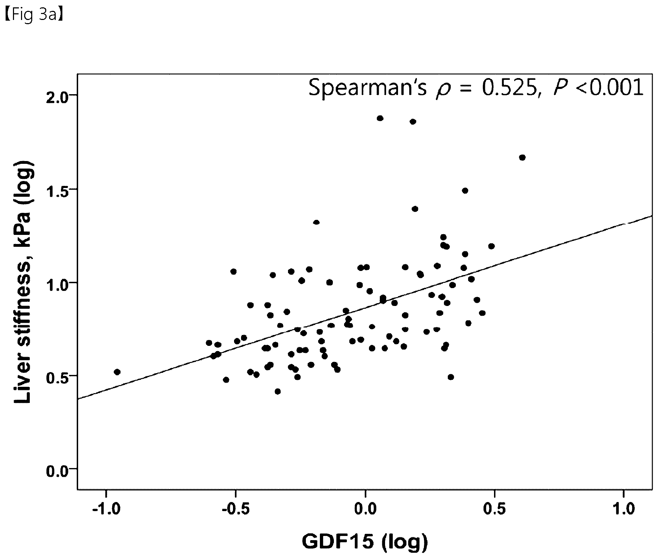

As can be confirmed in FIG. 3A, a significant positive correlation between GDF15 levels and liver stiffness was found (Spearman's ρ, 0.525; P <0.001).

Next, GDF15 levels were classified into four quartiles (Q), Q1 and Q4 being the lowest and highest quartiles, respectively. The prevalence of advanced fibrosis was 2.2%, 8.20, 8.50, and 41.7% in Q1, Q2, Q3, and Q4, respectively (P <0.001). The highest quartile (Q4; GDF15 level ≥ 1.52 ng/mL) of GDF15 was significantly associated with advanced fibrosis (unadjusted odds ratio (OR), 10.56; 95% confidence interval (CI), 4.35-25.60; P < 0.001).

### Result Example 3: Confirmation of GDF15 as determinant factor of fibrosis in NAFLD

It was subsequently investigated whether the risk of fibrosis progression could be restrictively predicted by means of GDF15 levels only in NAFLD patients.

As shown in Table 4, Multivariable Model 1 was adjusted for age, gender, and body mass index as variables. Additionally, Multivariable Model 2 was adjusted for smoking, hypertension, and diabetes in addition to the factors included in Multivariable Model 1; Multivariable Model 3 was adjusted for AST, platelet, and albumin in addition to the factors included in Multivariable Model 2; Multivariable Model 4 was adjusted for HOMA-IR in addition to the factors included in Multivariable Model 3; and Multivariable Model 5 was adjusted for LSMM in addition to the factors included in Multivariable Model 4.

**[Table 4]**

| | NASH | | Advanced fibrosis (≥F3) | |
|---|---|---|---|---|
| NAFLD (n=150) | OR (95% CI) | P-value | OR (95% CI) | P-value |
| Before adjustment | 1.79 (0.85, 3.76) | 0.123 | 9.18 (3.65, 23.12) | <0.001 |
| Age, gender adjusted | 1.94 (0.88, 4.27) | 0.099 | 6.39 (2.35, 17.39) | <0.001 |
| Multivariable Model 1 | 1.95 (0.88, 4.31) | 0.098 | 6.39 (2.35, 17.40) | <0.001 |
| Multivariable Model 2 | 1.87 (0.82, 4.27) | 0.135 | 6.56 (2.20, 19.61) | 0.001 |
| Multivariable Model 3 | 1.36 (0.47, 3.96) | 0.575 | 5.48 (1.71, 17.59) | 0.004 |
| Multivariable Model 4 | 1.53 (0.47, 5.02) | 0.480 | 4.39 (1.08, 17.90) | 0.039 |
| Multivariable Model 5 | 1.36 (0.39, 4.75) | 0.630 | 4.27 (1.04, 17.63) | 0.045 |

The GDF15 level in Q4 was significantly associated with advanced fibrosis, which remained significant in the analysis of multivariable models adjusted for age, gender, BMI, smoking status, diabetes, hypertension, AST levels, platelet counts, and albumin levels.

Additional adjustment for insulin resistance and LSMM showed statistical significance in the association between the GDF15 level and advanced fibrosis. In contrast, the GDF15 level in Q4 was not associated with the risk of NASH among NAFLD subjects.

However, it could be verified that GDF15 functions as an independent determinant factor in advanced fibrosis of NAFLD even after adjustment for LSMM and insulin resistance.

### Result Example 4: Correlation between presence or absence of diabetes and prevalence of advanced fibrosis

It was investigated whether GDF15 could have a decisive effect on advanced fibrosis regardless of the presence or absence of diabetes.

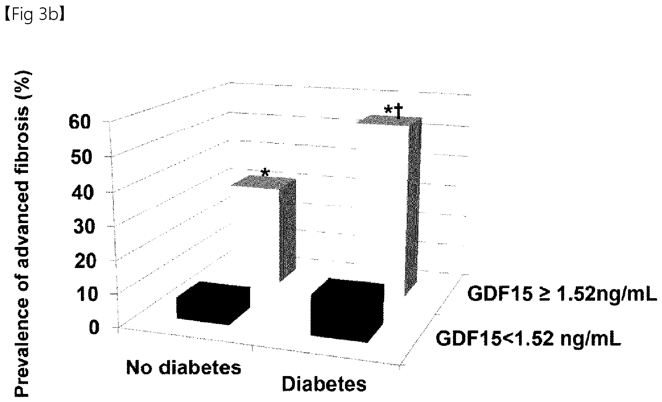

As can be confirmed in FIG. 3B, the results of stratified analysis using four quartiles of GDF15 levels showed additional differences for the severity of advanced fibrosis.

Especially, among subjects with diabetes, the prevalence of advanced fibrosis was 54.2% for GDF15 levels in Q4, and only 12.1% for GDF15 levels in Q1 to Q3. Particularly, when the GDF15 levels correspond to Q4, the prevalence of advanced fibrosis was 31.3% even in non-diabetic patients, indicating a 6-fold higher risk of advanced fibrosis compared with the risk of non-diabetic patients with GDF15 levels corresponding to Q1 to Q3 (OR, 6.72, 95% CI, 1.50-30.13).

The prevalence of advanced fibrosis associated with GDF15 levels was 29.8% (17/57) and 10.8% (10/93) in the NAFLD group, regardless of the presence or absence of diabetes (P = 0.003, chi-square test).

### Result Example 5: GDF15 treatment-induced fibrosis in LX-2 cells

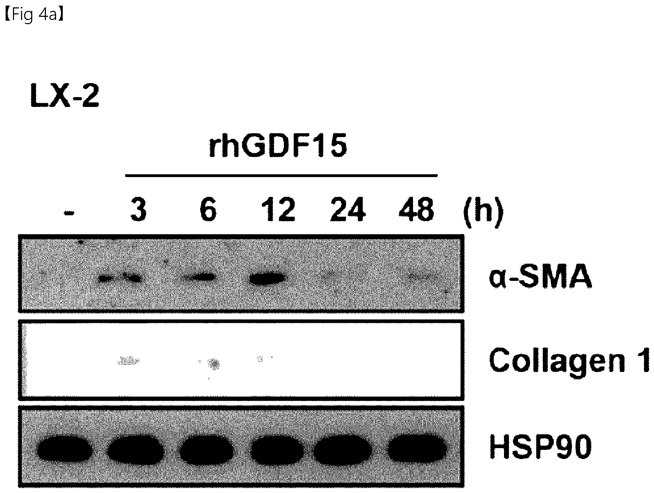

As can be confirmed in FIG. 4A, the expression of existing fibrosis markers, such as α-SMA and collagen 1, was increased in LX-2 cells by rhGDF15 treatment, and cell apoptosis was not influenced by the rhGDF15 treatment.

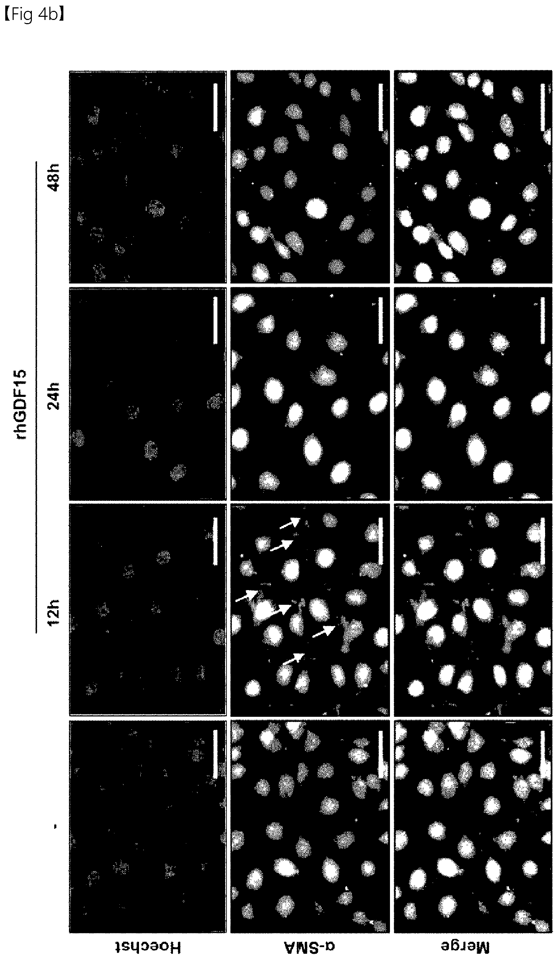

As can be confirmed in FIG. 4B, immunofluorescent staining results, similar to the immunoblotting results, also showed high expression of α-SMA in the GDF15 treatment for 12 hours, as shown in the portions indicated by the white arrows.

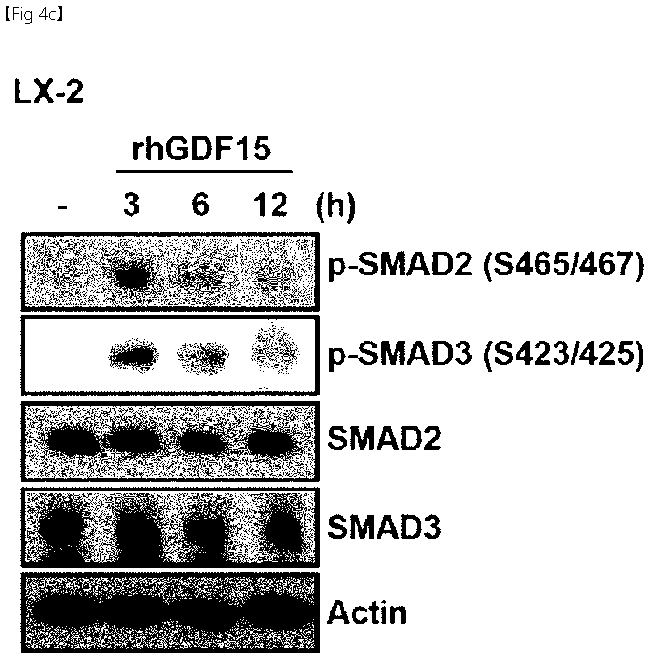

As can be confirmed in FIG. 4C, it was validated that GDF15 induced fibrosis by increasing the phosphorylation of SMAD2 and SMAD3 in human hepatocytes.

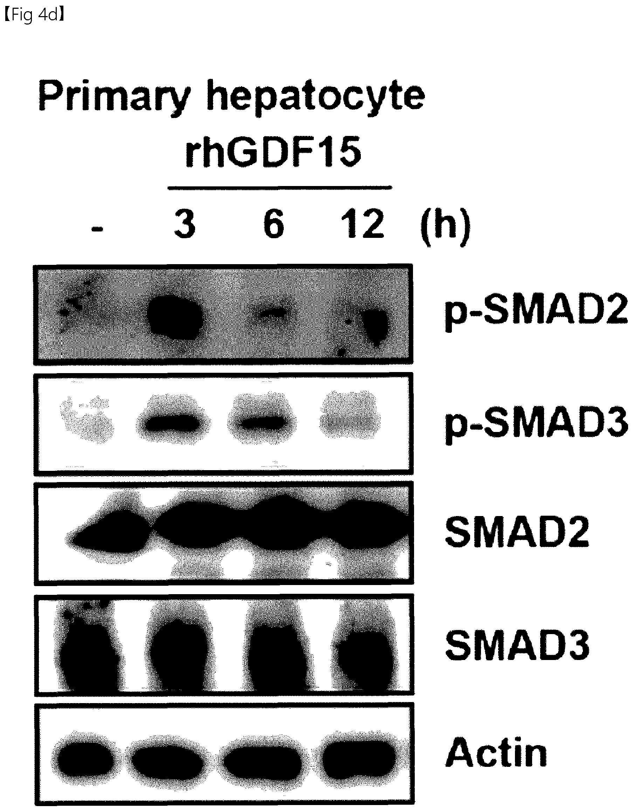

As can be confirmed in FIG. 4D, the GDF15 treatment induced fibrosis by upregulating the phosphorylation of SMAD2 and SMAD3 in primary hepatocytes.

The α-SMA level increased quickly within 12 hours after GDF15 treatment, and the SMAD phosphorylation was induced very quickly within 3 hours. It was revealed that GDF15 activates human hepatocytes and induces fibrosis since the phosphorylation of SMAD2 and SMAD3, known to play an important role in hepatocyte activation and fibrosis, was increased after the GDF15 treatment.

The acute stimulatory effect of GDF15 on hepatocytes was remarkably reduced after 12 hours, suggesting that GDF15 may be involved in the early response to liver injury or inflammation.

### Result Example 6: Increased GDF15 mRNA expression by palmitate treatment

It was investigated whether GDF15 can have a decisive effect on advanced fibrosis regardless of LSMM. Palmitate, known to induce muscle mass reduction, was applied to hepatocytes in a time- and dose-dependent manner, and GDF15 mRNA expression was assessed.

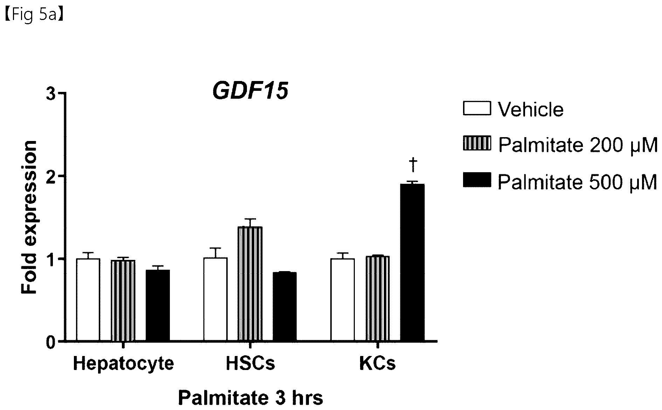

As can be confirmed in FIG. 5A, there is no change in hepatocytes after 3 hours of palmate treatment, and HSCs showed a slightly elevated expression level at 200 µM palmate, with no significance. However, Kupffer cells showed an approximately 2-fold significant increase in GDF15 mRNA expression level at 500 µM palmate (P = 0.004).

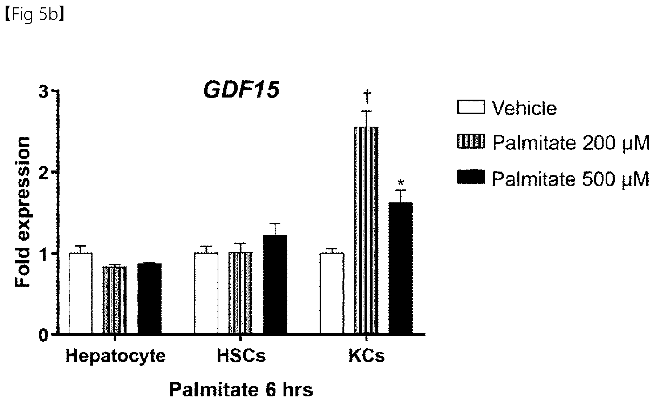

As can be confirmed in FIG. 5B, there were no significant changes in GDF15 mRNA expression levels in hepatocytes and HSCs after 6 hours of palmate treatment. The expression of GDF15 mRNA in Kupffer cells was increased 2.5-fold at 200 µM palmate (P = 0.009), and increased 1.5-fold at 500 µM palmate (P = 0.032).

Resultantly, it was verified that palmate treatment can increase the expression level of GDF15 mRNA in Kupffer cells, indicating that there is an inverse correlation between GDF15 expression and muscle mass.

### Industrial Applicability

The present invention relates to the use of a composition comprising an agent for measuring the expression of nucleic acid sequence encoding GDF15 protein or the activity of GDF15 protein, in a method for prediction or diagnosis of a liver disease and a method for prediction or diagnosis of a liver disease using the same and, more specifically, to the use of a marker in a method for prediction or diagnosis of liver fibrosis in nonalcoholic fatty liver disease and a method for prediction or diagnosis using the same.

## Claims

1. A method for prediction or diagnosis of a liver fibrosis in non-alcoholic fatty liver disease (NAFLD), the method comprising a measurement step of measuring the expression of a nucleic acid sequence encoding a growth differentiation factor 15 (GDF15) protein or the concentration of the GDF15 protein in a sample, wherein said expression or said concentration indicates the liver fibrosis.

2. The method of claim 1, wherein in the measurement step, it is determined whether the concentration of the GDF15 protein is 1.52 ng/mL or more.

3. The method of claim 1, wherein the GDF15 protein comprises the amino acid sequence of SEQ ID NO: 1.

4. A method for screening a candidate substance for prevention, treatment, or alleviation of a liver fibrosis in non-alcoholic fatty liver disease (NAFLD), the method comprising:
a treatment step of treating an analysis sample with a growth differentiation factor 15 (GDF15) protein and a treatment step of treating with the candidate substance; and
an analysis step of selecting an analysis sample inhibiting the expression of a nucleic acid sequence encoding a growth differentiation factor 15 (GDF15) protein or the concentration of the GDF15 protein; and
wherein the analysis sample comprises hepatocytes.

5. A method for screening a candidate substance for prevention, treatment, or alleviation of a liver fibrosis in non-alcoholic fatty liver disease (NAFLD), the method comprising:
a treatment step of treating an analysis sample with growth differentiation factor 15 (GDF15) protein-expressing cells and a treatment step of treating with the candidate substance; and
an analysis step of selecting an analysis sample inhibiting the expression of a nucleic acid sequence encoding a growth differentiation factor 15 (GDF15) protein or the concentration of GDF15 protein in the cells; and
wherein the analysis sample comprises hepatocytes.

## Patentansprüche

1. Verfahren zur Vorhersage oder Diagnose einer Leberfibrose bei nicht-alkoholischer Fettleberkrankheit (NAFLD), wobei das Verfahren einen Messschritt zum Messen der Expression einer Nukleinsäuresequenz, die für ein Protein des Wachstumsdifferenzierungsfaktors 15 (GDF15-Protein) kodiert, oder der Konzentration des GDF15-Proteins in einer Probe umfasst, wobei die Expression oder die Konzentration die Leberfibrose angibt.

2. Verfahren nach Anspruch 1, wobei in dem Messschritt bestimmt wird, ob die Konzentration des GDF15-Proteins 1,52 ng/ml oder mehr beträgt.

3. Verfahren nach Anspruch 1, wobei das GDF15-Protein die Aminosäuresequenz von SEQ ID NO: 1 umfasst.

4. Verfahren zum Screening einer Kandidatensubstanz zur Prävention, Behandlung oder Linderung einer Leberfibrose bei nicht-alkoholischer Fettleberkrankheit (NAFLD), wobei das Verfahren Folgendes umfasst:
einen Behandlungsschritt zum Behandeln einer Analyseprobe mit einem Protein des Wachstumsdifferenzierungsfaktors 15 (GDF15-Protein) und einen Behandlungsschritt zum Behandeln mit der Kandidatensubstanz; und
einen Analyseschritt zum Auswählen einer Analyseprobe, die die Expression einer Nukleinsäuresequenz, die für ein Protein des Wachstumsdifferenzierungsfaktors 15 (GDF15) kodiert, oder die Konzentration des GDF15-Proteins hemmt; und
wobei die Analyseprobe Hepatozyten umfasst.

5. Verfahren zum Screening einer Kandidatensubstanz zur Prävention, Behandlung oder Linderung einer Leberfibrose bei nicht-alkoholischer Fettleberkrankheit (NAFLD), wobei das Verfahren Folgendes umfasst:
einen Behandlungsschritt zum Behandeln einer Analyseprobe mit Zellen, die ein Protein des Wachstumsdifferenzierungsfaktors 15 (GDF15-Protein) exprimieren, und einen Behandlungsschritt zum Behandeln mit der Kandidatensubstanz; und
einen Analyseschritt zum Auswählen einer Analyseprobe, die die Expression einer Nukleinsäuresequenz, die für ein Protein des Wachstumsdifferenzierungsfaktors 15 (GDF15) kodiert, oder die Konzentration des GDF15-Proteins in den Zellen hemmt; und wobei die Analyseprobe Hepatozyten umfasst.

## Revendications

1. Méthode de prédiction ou de diagnostic d'une fibrose hépatique dans une stéatose hépatique non alcoolique (NAFLD), la méthode comprenant une étape de mesure destinée à mesurer l'expression d'une séquence d'acide nucléique codant une protéine du facteur de différenciation de croissance 15 (GDF15) ou la concentration de la protéine de GDF15 dans un échantillon, ladite expression ou ladite concentration indiquant la fibrose hépatique.

2. Méthode selon la revendication 1, dans laquelle dans l'étape de mesure, il est déterminé si la concentration de la protéine de GDF15 est supérieure ou égale à 1,52 ng/ml.

3. Méthode selon la revendication 1, ladite protéine de GDF15 comprenant la séquence d'acides aminés de la SEQ ID n° : 1.

4. Méthode de criblage d'une substance candidate pour la prévention, le traitement ou le soulagement d'une fibrose hépatique dans une stéatose hépatique non alcoolique (NAFLD), la méthode comprenant :
une étape de traitement destinée à traiter un échantillon d'analyse avec une protéine du facteur de différenciation de croissance 15 (GDF15) et une étape de traitement destinée à traiter avec la substance candidate ; et
une étape d'analyse destinée à sélectionner un échantillon d'analyse inhibant l'expression d'une séquence d'acide nucléique codant une protéine du facteur de différenciation de croissance 15 (GDF15) ou la concentration de la protéine de GDF15 ; et
ledit échantillon d'analyse comprenant des hépatocytes.

5. Méthode de criblage d'une substance candidate pour la prévention, le traitement ou le soulagement d'une fibrose hépatique dans une stéatose hépatique non alcoolique (NAFLD), la méthode comprenant :
une étape de traitement destinée à traiter un échantillon d'analyse avec des cellules exprimant une protéine du facteur de différenciation de croissance 15 (GDF15) et une étape de traitement destinée à traiter avec la substance candidate ; et
une étape d'analyse destinée à sélectionner un échantillon d'analyse inhibant l'expression d'une séquence d'acide nucléique codant une protéine du facteur de différenciation de croissance 15 (GDF15) ou la concentration de la protéine de GDF15 dans les cellules ; et ledit échantillon d'analyse comprenant des hépatocytes.
